# EUROPÄISCHE PATENTSCHRIFT

(11) **EP 2 674 189 B1**
(45) Veröffentlichungstag und Bekanntmachung des Hinweises auf die Patenterteilung: **20.04.2016**
(21) Anmeldenummer: 12004397.1
(22) Anmeldetag: 11.06.2012
(51) Int. Cl.: A61N 1/05

(54) **Implantierbarer Elektroden Pol**
Implantable electrode pole
Pôle d'électrodes implantables

(43) Veröffentlichungstag der Anmeldung: 18.12.2013
(73) Patentinhaber: Peter Osypka Stiftung, 79639 Grenzach-Wyhlen (DE)
(72) Erfinder: Osypka, Peter, 79639 Grenzach-Wyhlen (DE)

(56) Entgegenhaltungen:
- DE-U1-202010 011 492
- DE-U1-202011 108 639
- US-A- 5 855 592
- US-A1- 2008 082 132
- US-B1- 7 450 999
- US-B1- 7 515 971

## Beschreibung

Die Erfindung betrifft einen implantierbaren Elektroden Pol, der für alle Kammern des Herzens, sowohl für die Stimulation, Kardioversion und auch für die Defibrillation, eingesetzt werden kann.

### Problem und die bisherige Lösung

Implantierbare Herzschrittmacher Elektroden gibt es seit über 60 Jahren des vorigen Jahrhunderts, also der Zeit in der der erste Herzschrittmacher implantiert wurde.

Die hohe Bruchsicherheit der Elektroden Zuleitung zwischen Herzschrittmacher und Herz und die gute Fixierbarkeit der Elektrode in der rechten Herzkammer haben seit den 70er Jahren weltweit zur großen Anwendung der Herzschrittmacher Therapie beigetragen.

Nach wie vor lässt die Elektroden Platzierung in der linken Herzseite, sowohl im Atrium als auch im linken Ventrikel zu wünschen übrig. Für die Stimulation des linken Ventrikels nimmt man den Koronar Sinus als Elektrodenplatzierung zur Hilfe. Für die permanente Stimulation des linken Vorhofes gibt es bisher noch keine einfache Lösung.

Dokument DE 20 2010 011 492 U1 beschreibt eine Herzkatheter zur Fixierung im Septum, wobei die Fixierung im rechten Vorhof über eine geflochtene doppelwandige Nitinolscheibe erfolt und der distale Teil des Katheters auch als indifferent Elektrode benutzt werden kann.

### Lösungsweg:

Die Lösung ist ein großflächiger indifferente Elektroden Pol, der eine elektrische Stimulation der Vorhöfe des Herzens, insbesondere des linken Vorhofs, also ein kontinuierliches "Multisite Pacing" ermöglicht. Das geschieht mit einem Elektroden Pol im Septum zwischen den Vorhöfen des Herzens, der zunächst für beide Vorhöfe des Herzens als indifferente Elektrode, zusammen mit einem implantierbaren Herzschrittmacher, wirksam ist. Als differente Elektroden, können weiterhin die üblichen Standard Herzschrittmacher Elektroden verwendet werden. Darüber hinaus kann diese indifferente Elektrode auch als Pol für die Defibrillation bei Kammerflimmern verwendet werden. Durch eine variable Öffnung in der Elektrode wird ein Katheter Zugang zum linken Vorhof und auch zum linken Ventrikel möglich, was bei der Ablation von Vorhofflimmern und bei einem Austausch von Herzklappen vorteilhaft ist.

Die Erfidnung ist in Anspruch 1 definiert, weitere Merkmale und Ausführungsbeispiele sind in den abhängigen Ansprüchen definiert.

### Zusammenfassung der Vorteile:

Mit dieser Erfindung werden folgende Vorteile möglich:
1. Eine Vorhof Stimulation des Herzens, dass das Auftreten von Vorhofflimmern verhindert und damit die Schlaganfall Gefährdung erheblich reduziert und die Belastung der Patienten vermindert.
2. Eine ideale Position eines Pols bei einer Defibrillation (Kammerflimmern) oder Kardioversion (Vorhofflimmern).
3. Ein schneller Zugang zum linken Vorhof und - Ventrikel.

### Darstellung der Abbildungen

Weitere Einzelheiten, Merkmale und Vorteile der Erfindung lassen sich dem folgenden Beschreibungsteil entnehmen, in dem anhand der Zeichnungen und Ausführungsbeispiele die Erfindung näher erläutert wird. Sie zeigen in schematischer Darstellung in
Fig. 1 eine beispielhafte Darstellung des Elektroden Pols im atrialen Septum des Herzens.
Fig. 2 eine beispielhafte Darstellung des Elektroden Pols im atrialen Septum und einer Schraub Elektrode im linken Vorhof des Herzens.
Fig. 3 eine beispielhafte Darstellung des Elektroden Pols im atrialen Septum, der auch als Zugang eines Katheters in den linken Ventrikel des Herzens ermöglicht.
Fig. 4 eine beispielhafte Darstellung des Elektroden Pols im atrialen Septum des Herzens, mit üblichen Elektroden in und um das Herz.
Fig. 5 eine beispielhafte detaillierte Darstellungen des Elektroden Pols im atrialen Septum des Herzens mit einer elektrischen Verbindung zu einem Herzschrittmacher in Form einer Wendel.
Fig. 6 eine weitere beispielhafte detaillierte Darstellungen des Elektroden Pols im atrialen Septum des Herzens mit einer elektrischen Verbindung zum Herzschrittmacher in Form einer Wendel.
Fig. 7 eine weitere beispielhafte detaillierte Darstellungen des Aufbaus eines Elektroden Pols mit einer dehnbaren Inneren Öffnung.
Fig. 8 weitere beispielhafte detaillierte Darstellungen des Aufbaus eines Elektroden Pols.
Fig. 9 weitere beispielhafte detaillierte Darstellungen des Aufbaus eines Elektroden Pols mit unterschiedlichen Fixierungsarten.

### Beschreibung der Abbildungen:

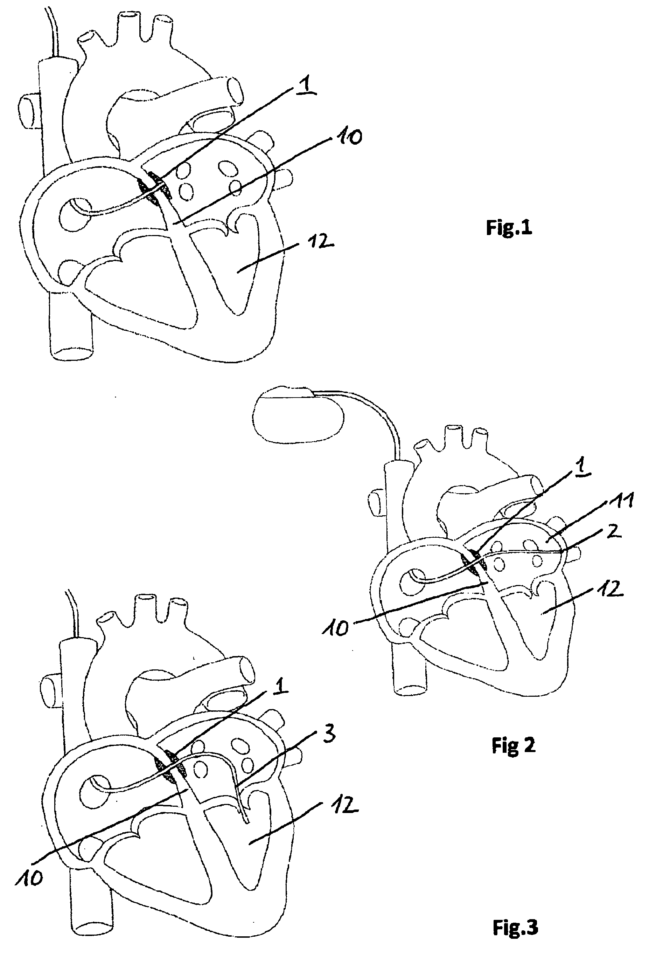

Fig. 1 zeigt eine beispielhafte Darstellung des Elektroden Pols (1) im atrialen Septum (10) des Herzens. Die Platzierung des Pols erfolgt ähnlich wie bei den PFO- oder ASD Occludern. Die elektrische Verbindung kann zum Beispiel in die vena subclavia münden um dort später an einen Herzschrittmacher angeschlossen zu werden.

Fig. 2 zeigt eine beispielhafte Darstellung des Elektroden Pols(1) im atrialen Septum(10) und einer Schraub Elektrode(2) im linken Vorhof(11) des Herzens. Hier dient der Pol sowohl als indifferente Elektrode als auch als Durchgang für die Schraub Elektrode. Zwischen diesen beiden Polen erfolgt die Stimulation des linken Vorhofs. Als Elektrode für den linken Vorhof ist hier eine Schraub Elektrode dargestellt, die seit 35 Jahren routinemäßig als implantierbare Herzschrittmacher Elektrode zur Anwendung kommt. Schraubelektroden eignen sich besonders gut für die Fixierung in den Vorhöfen des Herzens, da sie bei sorgfältiger Platzierung nicht dislozieren.

Fig. 3 zeigt eine beispielhafte Darstellung des Elektroden Pols(1) im atrialen Septum(10), der auch den Zugang eines Katheters(3) in den linken Ventrikel(12) des Herzens ermöglicht. Hier dient die zentrale Öffnung des Pols als Zugang von Kathetern oder Vorrichtungen in die linke arterielle Seite des Herzens.

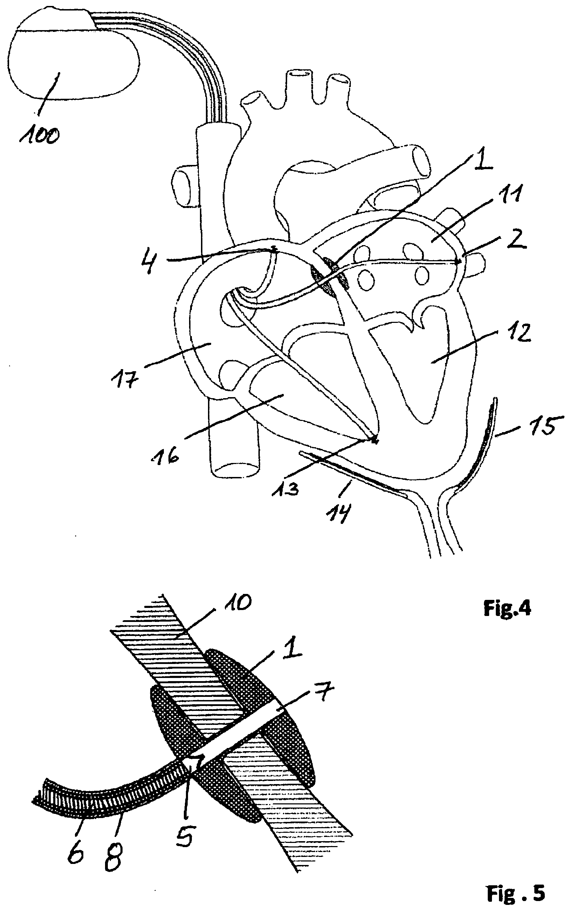

Fig. 4 zeigt eine beispielhafte Darstellung des Elektroden Pols(1) im atrialen Septum(10), der als indifferente Elektrode, zusammen mit den heute üblichen implantierbaren Herzschrittmacher Elektroden, alle vier Herzkammem stimulieren und defibrillieren kann.

Stimulation des rechten Vorhofs(17): Elektroden Position zwischen rechten Vorhof (4) und des Elektroden Pols(1).

Stimulation des rechten Ventrikels(16): Elektroden Position zwischen rechten Ventrikel (13) und des Elektroden Pols(1).

Stimulation des linken Vorhofs(11): Elektroden Position zwischen linken Vorhof (2) und des Elektroden Pols(1).

Stimulation des linken Ventrikels(12): Elektroden Position zwischen Elektrode(15) und des Elektroden Pols(1).

Defibrillation des gesamten Herzens: Elektroden Position zwischen den Elektroden (14,15) und des Elektroden Pols (1).

Kardioversion bei Vorhofflimmern: Elektroden Position zwischen den Elektroden (4,2) und des Elektroden Pols (1).

Fig. 5 zeigt eine detaillierte Darstellung des Elektroden Pols (1) im atrialen Septum(10) mit der elektrischen Verbindung zu einem Stimulator/Defibrillator in Form einer Wendel (6) mit entsprechender Silikon- oder Polyurethan Isolierung (8). Die Wendel dient gleichzeitig als ein Führungskatheter der den Zugang in die linke Herzhälfte ermöglicht. Der proximale Teil der Wendel führt über eine Vene (z.B. subclavia) in das "Schrittmacher Bett". Dort kann die Zuleitung an einen genormten IS1-Stecker für Herzschrittmacher/Defibrillator (100) oder mit einem Port-System verbunden werden, das einen späteren Zugang zum linken Vorhof ermöglicht. Das Drahtgeflecht des Elektroden Pols, das gewöhnlich innerhalb von ein paar Wochen im Septum einwächst, hat im Zentrum eine Öffnung (7) mit einem Ventil (5), das einmal den Blutfluss vom linken in den rechten Vorhof verhindert, andererseits den Durchgang eines Katheters oder Elektrode zur Stimulation des linken Vorhofs (11) ermöglicht, oder einen Zugang für einen Katheter in die linke Herzkammer(12) schafft. Das Ventil (5), das vorzugshalber aus Silikon aufgebaut ist, enthält eine Medikament (z.B. Paclitaxel), das verhindert, dass der Zugang sich durch Gewebe Bildung verschließt. Die hier dargestellte zentrale Öffnung(7) des Elektroden Pols, kann bei Bedarf auch an anderer Stelle im Drahtgeflecht angebracht werden. Damit wird ein einfacher Zugang sowohl zum linken Vorhof als auch zum linken Ventrikel des Herzens gewährleistet. Das ist besonders vorteilhaft bei Operationen an der Mitralklappe und bei der RF-Ablation im linken Vorhof und den Pulmonal Venen. Die elektrische Stimulation erfolgt demnach beispielsweise zwischen den implantierten Elektroden in den Vorhöfen (2und 4) und dem gemeinsamen indifferenten Elektroden Pol (1) im Septum.

Durch die jetzt geschaffene Möglichkeit der gleichzeitigen elektrischen Stimulation beider Vorhöfe, dem sogenannten "multisite Pacing", könnte das Auftreten von Vorhofflimmern verhindert werden. Da die Häufigkeit des Vorhofflimmerns bei jedem Patienten unterschiedlich ist, kann die Intensität, Dauer und Wiederholung der Stimulation, durch die Programmierung des Schrittmachers individuell eingestellt werden. So könnte beispielsweise, um das Auslösen des Vorhofflimmerns zu unterdrücken, die Stimulation nur stündlich für ein paar Minuten notwendig sein. Die Implantation von Elektroden in dem rechten Vorhof ist bekannt und wird seit vielen Jahren routinemäßig eingesetzt.

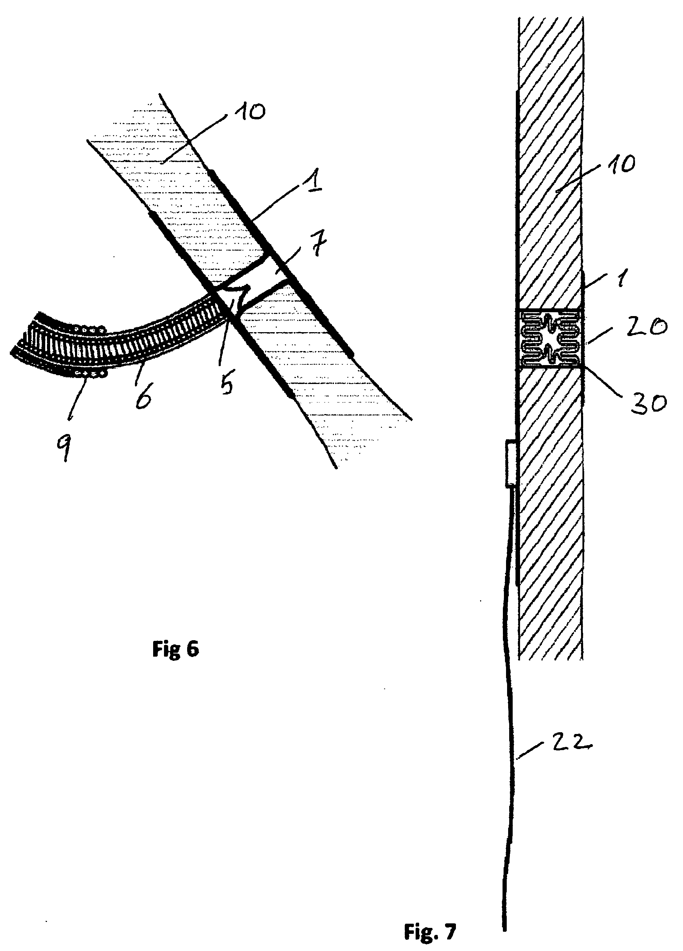

Fig. 6 zeigt eine weitere beispielhafte detaillierte Darstellung des Elektroden Pols (1). Der Zugang zum linken Vorhof (11) erfolgt durch die bekannte transseptale Punktion (hier nicht dargestellt). In die durch die Punktion erfolgte Öffnung im Septum wird ein aus Nitinol Draht geflochtener Schirm implantiert, der als Elektroden Pol (1) wirksam ist und mit einer elektrischen Verbindung (6) mit einem Herzschrittmacher/Defibrillator (100) verbunden werden kann. Der zweite Pol stellt entweder das Schrittmacher Gehäuse dar oder er ist auf einer zweiten äußeren Wendel (9) angebracht. Je nach Aufbau kann der geflochtene Schirm(1) aus einem Einfach Geflecht (Fig.6) oder aus einem Doppel Geflecht (Fig.5) bestehen.

Fig. 7 zeigt eine weitere beispielhafte detaillierte Darstellung des Elektroden Pols (1) im atrialen Septum (10). Die Öffnung im Zentrum (20) ist als Stent ausgebildet(30) und kann bei Bedarf mit Hilfe eines Ballon Katheters gedehnt werden. Der hier dargestellte Pol (1) besteht aus zwei einfach geflochtenen Schirmen. Die elektrische Verbindung des Pols (1) mit einem Schrittmacher/Defibrillator (hier nicht dargestellt) erfolgt über ein Kabel (22).

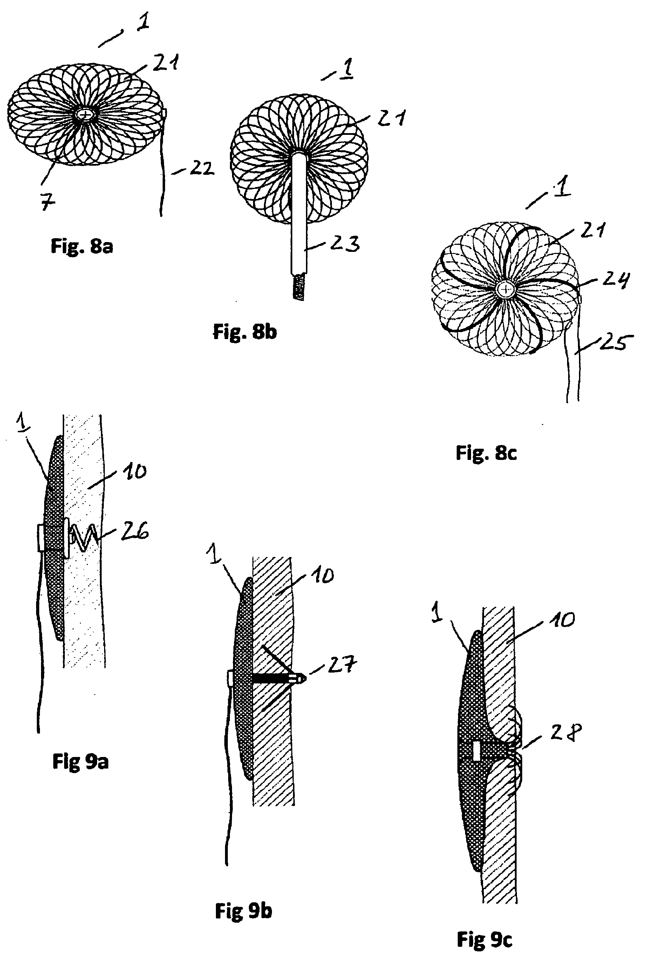

Fig. 8a-c zeigen beispielhafte detaillierte Ansichten des Elektroden Pols (1). Er besteht aus einer Anzahl von Nitinol Drähten (21), die zu einem Schirm mit unterschiedlicher Form geflochten sind und elektrisch mit einem Kabel (22) verbunden ist. Im Zentrum befindet sich eine Öffnung (7)(Fig.8a), die als Zugang zum linken Herzen genutzt werden kann oder es existiert eine direkte Schaft Verbindung (23) (Fig. 8b) zu einem Stimulator/Defibrillator (hier nicht dargestellt). Jeder Schirmteil kann als Einfach- oder Doppelgeflecht in unterschiedlicher Größe und bei Bedarf teilweise(24, Fig. 8c) isoliert ausgeführt werden. Damit entsteht eine bipolare Anordnung, die über die beiden Kabel (25) mit einem Stimulator verbunden werden.

Fig. 9a-c zeigen beispielhafte Ansichten der Fixierung des Elektroden Pols (1) im Septum (10). Die Fixierung kann mit Hilfe einer Schraubwendel (26, Fig.9a) oder mittels eines dehnbaren Spreiz Hakens (27, Fig. 9b) erfolgen. Eine weitere Möglichkeit besteht darin, dass die Draht Enden des Schirm Geflechtes so geformt werden, dass sie nach Verlassen des Einführungs Katheters sich nach Innen in das Septum verhaken (28, Fig.9c). Auch kann eine Seite oder auch beide Seiten des Poles mit einer biokompatiblen Kunstsoff Schicht, vorzugshalber Silikon oder Polyurethan, bedeckt werden.

## Patentansprüche

1. Implantierbarer Elektroden Pol (1), der nach Bedarf in Verbindung mit einem Herzschrittmacher (HSM) oder einem Defibrillator (ICD) eingesetzt werden kann, bestehend aus einer grossflächigen indifferenten Elektrode (1), geeignet zum Implantieren im atrialen Septum und einer elektrischen Verbindung (6),(22), (23) mit der die Elektrode an den Herzschrittmacher/ Defibrillator angeschlossen werden kann, **dadurch gekennzeichnet, dass** die Elektrode aus mindestens einem Kreis förmigen oder Ellipsen förmigen Metallgeflecht oder Metallgewebe, vorzugsweise aus Nitinol, besteht und dass die Elektrode mindestens eine Öffnung (7) (20) enthält, die einen Zugang in den linken Vorhof gestattet und die mit einem flexiblen Schaft (23) indem die elektrische Verbindung verläuft, verbunden werden kann und dass die Öffnung (7) ein Ventil (5) enthält.

2. Implantierbarer Elektroden Pol nach Anspruch 1, **dadurch gekennzeichnet, dass** die innere Öffnung ein Ventil enthält, das eine medikamentenfreisetzende Substanz enthält.

3. Implantierbarer Elektroden Pol nach Anspruch 1, **dadurch gekennzeichnet, dass** der innere Aufbau des Schaftes (23) aus mindestens einer Metallwendel (6) besteht.

4. Implantierbarer Elektroden Pol nach Anspruch 1,**dadurch gekennzeichnet, dass** der innere Aufbau des Schaftes (23) aus einem Metallgeflecht besteht.

5. Implantierbarer Elektroden Pol nach Anspruch 1 bis 4, **dadurch gekennzeichnet, dass** der proximale Anschluss des Schaftes (23) schlauchförmig aufgebaut ist, sodass er mit einem Infusions Port verbunden werden kann und als Kanal nutzbar ist oder als Zugang zum linksseitigen arteriellen System des Herzens genutzt werden kann.

6. Implantierbarer Elektroden Pol nach Anspruch 1,**dadurch gekennzeichnet, dass** die elektrische Verbindung (22) zum Elektroden Pol am äusseren Rand des Geflechtes angebracht ist.

7. Implantierbarer Elektroden Pol nach Anspruch 1 bis 6, **dadurch gekennzeichnet, dass** mindestens eine Seite des Elektrodenpols mit einem biokompatiblen Kunststoff beschichtet ist.

## Claims

1. An implantable electrode pole (1) which can be used in connection with a pacemaker (PM) or a defibrillator (DCI), consisting of large-area indifferent electrode (1), being suitable for implantation in the atrial septum and an electrical connection (6), (22), (23) with which the electrode can be connected to the pacemaker/defibrillator, **characterized in that** the electrode is composed of at least one circular or elliptical metallic braiding or mesh, preferably made of Nitinol, and that the electrode has at least one opening (7) (20), which allows an access to the left atrium and which can be connected to a flexible shaft (23) in which the electrical connection runs, and that the opening (7) has a valve (5).

2. An implantable electrode pole according to claim 1, **characterized in that** the inner opening has a valve, containing a medicament releasing substance.

3. An implantable electrode pole according to claim 1, **characterized in that** the inner assembly of the shaft (23) consists of at least one metallic coil (6).

4. An implantable electrode pole according to claim 1, **characterized in that** the inner assembly of the shaft (23) consists of a metallic mesh.

5. An implantable electrode pole according to claim 1 to 4, **characterized in that** the proximal terminal of the shaft (23) has a tubular construction, so that it can be connected with an infusion port and is available as a channel or can be used as an access to the left arterial system of the heart.

6. An implantable electrode pole according to claim 1, **characterized in that** the electrical connection (22) is mounted to the electrode pole at the outer periphery of the braiding.

7. An implantable electrode pole according to claim 1 to 6, **characterized in that** at least one side of the electrode pole is coated with a biocompatible plastic.

## Revendications

1. Un pôle d'électrode implantable (1), qui si besoin peut être connecté à un pacemaker (PM) ou à un défibrillateur (DCI), constitué d'une électrode indifférente à grande surface (1), adaptée à être implantée dans la septum auriculaire et une liaison électrique (6), (22), (23) avec laquelle l'électrode peut être connectée au pacemaker/défibrillateur, **caractérisé en ce que** l'électrode est composée d'au moins un treillis ou tissu métallique de forme ronde ou elliptique, préférentiellement en Nitinol, et **en ce que** l'électrode possède au moins une ouverture (7) (20), qui permet un accès dans l'oreillette gauche et qui peut être connecté avec une gaine souple (23) dans laquelle une liaison électrique s'étend, et que l'ouverture (7) possède une soupape (5).

2. Un pôle d'électrode implantable selon la revendication 1, **caractérisé en ce que** l'ouverture intérieure comprenne une soupape, contenant une substance libérant des médicaments.

3. Un pôle d'électrode implantable selon la revendication 1, **caractérisé en ce que** la structure interne de la gaine (23) est composée d'au moins une spirale métallique (6).

4. Un pôle d'électrode implantable selon la revendication 1, **caractérisé en ce que** la structure interne de la gaine (23) est composée d'un treillis métallique.

5. Un pôle d'électrode implantable selon les revendications 1 à 4, **caractérisé en ce que** le raccordement proximal de la gaine (23) est tubulaire, afin qu'il puisse être connecté avec un raccord d'infusion et être utilisé en tant que conduit ou accès au système artériel gauche du coeur.

6. Un pôle d'électrode implantable selon la revendication 1, **caractérisé en ce que** la liaison électrique (22) du pôle de l'électrode est placée à la périphérie extérieure du treillis.

7. Un pôle d'électrode implantable selon les revendications 1 à 6, **caractérisé en ce que** au minimum un côté du pôle de l'électrode est revêtu d'une matière synthétique biocompatible.
